# EUROPEAN PATENT APPLICATION

(11) **EP 4 825 953 A1**
(43) Date of publication of application: **21.10.2026**
(21) Application number: 23961481.1
(22) Date of filing: 14.12.2023
(51) Int. Cl.: C12M 1/00

(54) **LIQUID RECOVERY DEVICE**

(71) Applicant: Hitachi High-Tech Corporation, Minato-ku Tokyo 105-6409 (JP)
(72) Inventor: FUJIOKA, Kaoru, Tokyo 105-6409 (JP); FUJIOKA, Michiru, Tokyo 105-6409 (JP); SUMIDA, Noriyuki, Tokyo 105-6409 (JP)
(74) Representative: MERH-IP Matias Erny Reichl Hoffmann Patentanwälte PartG mbB
(86) International application number: PCT/JP2023/044816
(87) International publication number: WO 2025/126416

(57) **Abstract**

This liquid recovery device comprises: a magnetic rod that attracts magnetic beads; a first container that accommodates an extraction liquid for extracting a nucleic acid, and the magnetic beads, which are capable of adsorbing and releasing the nucleic acid; and a recovery region that is for recovering the extraction liquid, as a residual liquid, from the magnetic rod while the magnetic beads are magnetically adhered thereto. The liquid recovery device extracts the nucleic acid in the first container, and then brings the magnetic rod, which is in a condition in which the magnetic beads are magnetically adhered thereto, into contact with the recovery region, and recovers the residual liquid.

## Description

### Technical Field

The present invention relates to a liquid recovery device, and more particularly to a liquid recovery device used for extracting nucleic acids from a sample containing nucleic acids and magnetic beads.

### Background Art

In recent years, information obtained by nucleic acid analysis such as genomic testing using a next-generation sequencing (NGS) system has been used in various fields such as medical care, clinical testing, pharmaceutical industries, and food industries. In such nucleic acid analysis, nucleic acid extraction from various biological samples such as blood, tissue, and a cultured cell is essential preprocessing.

As nucleic acid extraction methods, a method based on a property that nucleic acids bind to silica under the presence of a chaotropic agent and a method based on a property that the nucleic acids bind to silica under the presence of an organic solvent are generally used instead of methods using phenol, chloroform, and the like, which are hazardous organic solvents. Using these methods, a nucleic acid extraction method using a nucleic acid capturing tip including a solid phase containing silica as a nucleic acid capturing carrier, and a method using magnetic beads (nucleic acid capturing carrier) whose surface is covered with silica have been used. These methods include a step of binding nucleic acids to a nucleic acid capturing carrier, a cleaning step of removing impurities, and an extraction step of eluting nucleic acids from a nucleic acid capturing carrier using an extraction liquid (also referred to as an eluent, an eluate, or the like).

In the method using magnetic beads, the magnetic beads are separated using a magnet from a mixed liquid of the extraction liquid from which the nucleic acids are extracted and the magnetic beads, and the extraction liquid is recovered. As an example, there is a method of aspirating the extraction liquid containing the magnetic beads into a dispensing tip, retaining the magnetic beads in the dispensing tip using the magnet, and discharging only the extraction liquid from the dispensing tip. As another example, there is a method of recovering magnetic beads from an extraction liquid containing the magnetic beads by inserting a rod-shaped magnet (which may be covered with a cover) into the extraction liquid.

PTL 1 discloses, as a method for efficiently collecting magnetic beads, a method of collecting magnetic beads on a wall of a container first and then recovering the magnetic beads using a magnetic rod.

PTL 2 discloses a technique of implementing collection of a magnetic body from a magnetic body containing liquid not on a side of a container containing a sample but using a dispenser that aspirates and discharges the magnetic body containing liquid.

PTL 3 discloses a technique of preventing scattering of a liquid and an aerosol by designing a shape of a cover of a magnetic body for collecting magnetic beads into a shape for covering an outside of a reaction container in addition to a function for covering the magnetic body.

### Citation List

### Patent Literature

PTL 1: US Patent No. 6020211
PTL 2: JPH11-262678A
PTL 3: WO2014/007074A

### Summary of Invention

### Technical Problem

The testing using nucleic acids of a biological sample is usually performed in the order of "collection of a biological sample", "nucleic acid extraction from the biological sample", and "nucleic acid analysis". The present invention solves the problem of "nucleic acid extraction from the biological sample".

First, a nucleic acid extraction method using magnetic beads and a magnetic rod will be described. In the final step of nucleic acid extraction, when a magnetic rod, which is a rod-shaped magnet, is inserted into an extraction liquid containing magnetic beads, the magnetic beads in the liquid is collected on the magnetic rod. This is called "magnetism collection" in the present invention. At this time, it is desirable that the magnetic rod is covered with a cover made of a material such as plastic, and in this case, the magnetic beads are collected on the magnetic rod through the cover. When the magnetic rod and the cover are removed from a container, the magnetic beads and the extraction liquid are separated from each other, and a nucleic acid extraction liquid remains in the container. This nucleic acid extraction liquid is used for the following "nucleic acid analysis" such as NGS and dPCR.

In recent years, in order to improve the measurement sensitivity of nucleic acid analysis, it is required to extract as many nucleic acids as possible and use a large amount of nucleic acids for nucleic acid analysis. For this purpose, it is necessary to performing extraction with a small amount of nucleic acid extraction liquid from a larger amount of biological samples than in the related art. As the amount of the biological samples increases, a larger amount of nucleic acids are given. In addition, since an amount of nucleic acid extraction liquid used for nucleic acid analysis such as NGS after nucleic acid extraction is limited in each nucleic acid analysis method, there is an effect that the nucleic acid extraction liquid is concentrated by performing extraction with a small amount of nucleic acid extraction liquid, and more nucleic acids than in the related art can be used for nucleic acid analysis.

When the amount of the biological samples to be treated increases, it is necessary to increase the amount of the magnetic beads as the nucleic acid capturing carrier according to the amount. When the magnetic rod and the cover are removed from the container after magnetism collection, not only the magnetic beads but also a slight amount of the extraction liquid adheres to the magnetic rod or the cover, resulting in loss of the extraction liquid. In the present invention, the extraction liquid adhering to the magnetic rod or the cover is referred to as a "residual liquid". In particular, it was found that when the amount of magnetic beads increased, the volume of the magnetic beads collected on the magnetic rod and the cover increased, and at the same time, a loss amount of the extraction liquid (that is, residual liquid) increased. Further, in the extraction with a small amount of nucleic acid extraction liquid, since an absolute amount of the extraction liquid is small, the influence of loss becomes relatively large.

An object of the present invention is to provide an extraction liquid recovery device capable of reducing loss during separation of magnetic beads and an extraction liquid in a nucleic acid extraction method using magnetic beads and a magnetic rod.

### Solution to Problem

An example of a liquid recovery device according to the present invention is a liquid recovery device including:
a magnetic rod that adsorbs magnetic beads;
a first container that accommodates an extraction liquid for extracting nucleic acids, and the magnetic beads configured to adsorb and release the nucleic acids; and
a recovery region for recovering the extraction liquid as a residual liquid from the magnetic rod in a state of adsorbing the magnetic beads, in which
the liquid recovery device extracts the nucleic acids in the first container, then brings the magnetic rod in the state of adsorbing the magnetic beads into contact with the recovery region, and recovers the residual liquid.

### Advantageous Effects of Invention

According to a liquid recovery device of the present invention, even if a residual liquid is present on a magnetic rod or a cover to which magnetic beads adhere, the residual liquid can be recovered by contact. Accordingly, since a larger amount of residual liquid can be recovered than in the related art, a larger amount of nucleic acids can be extracted, and a larger amount of nucleic acids can be used for nucleic acid analysis.

### Brief Description of Drawings

[FIG. 1] FIG. 1 is a diagram illustrating a nucleic acid extraction method using magnetic beads 1 and a magnetic rod 2 according to the related art.
[FIG. 2] FIG. 2 illustrates a result obtained by experimentally examining an influence of the amount of the magnetic beads 1 in an extraction step on a recovery amount of an extraction liquid in the related art.
[FIG. 3A] FIG. 3A is a schematic diagram illustrating an extraction step in the case of using a normal amount of the magnetic beads 1 in an experiment in FIG. 2.
[FIG. 3B] FIG. 3B is a schematic diagram illustrating an extraction step in the case of using the magnetic beads 1 in the amount of 5 times in the experiment in FIG. 2.
[FIG. 4] FIG. 4 illustrates an example of a liquid recovery device including a first container 10 and an empty second container 11 according to Embodiment 1 of the present invention.
[FIG. 5] FIG. 5 illustrates a result of an experiment for verifying an effect of additional collection in Embodiment 1.
[FIG. 6] FIG. 6 illustrates an operation diagram when the magnetic rod 2 to which a residual liquid 9 adheres is brought into contact with the second container 11 in Embodiment 2.
[FIG. 7] FIG. 7 illustrates an example of a liquid recovery device including a contact portion 13 for recovering the residual liquid 9 in a part of the first container 10 according to Embodiment 3.
[FIG. 8] FIG. 8 illustrates a specific example of replacement of a magnetic rod.
[FIG. 9] FIG. 9 illustrates a specific example of adjustment of a distance between the magnetic rod and magnetic beads.

### Description of Embodiments

Hereinafter, a nucleic acid extraction method using a liquid recovery device in the related art will be described. A part of the related art described below is also used in an embodiment of the present invention.

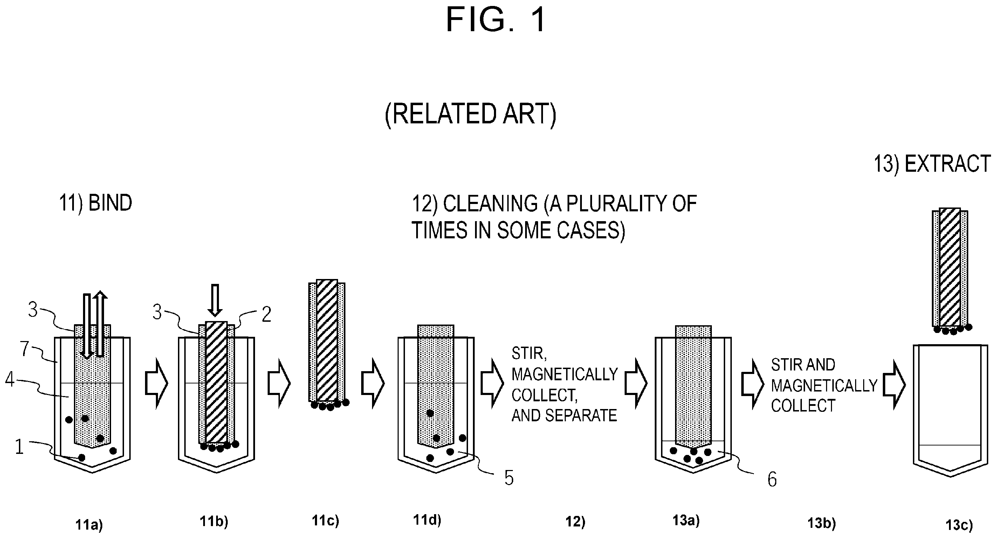

FIG. 1 is a diagram illustrating a nucleic acid extraction method using magnetic beads 1 and a magnetic rod 2 according to the related art. The magnetic rod 2 may include a cover 3 that covers the magnetic rod 2. The magnetic rod 2 has a function of collecting the magnetic beads 1. When the cover 3 is not provided, the magnetic beads 1 are directly collected on the magnetic rod 2. When the magnetic rod 2 is covered with the cover 3, the magnetic beads 1 are collected on the magnetic rod 2 through the cover 3.

Hereinafter, in the present description, even when simply described as the magnetic rod 2, it may mean a combination of the magnetic rod 2 and the cover 3.

The role of the cover 3 is used for the purpose of separating the magnetic rod 2 and the magnetic beads 1 or protecting the magnetic rod 2 when the magnetic beads 1 magnetically collected in the magnetic rod 2 are diffused into a solution (for example, a binding liquid 4, a probe cleaner 5, or an extraction liquid 6).

In the nucleic acid extraction method using the magnetic beads 1 and the magnetic rod 2, the following operation is performed.
"Stirring" of the binding liquid 4, the probe cleaner 5, or the extraction liquid 6 by the vertical movement of the cover 3
"Magnetism collection" in which the magnetic rod 2 is inserted into the binding liquid 4, the probe cleaner 5 or the extraction liquid 6 containing the magnetic beads 1 to collect the magnetic beads 1 on the magnetic rod 2
"Separation" of the magnetic beads 1 from the binding liquid 4, the probe cleaner 5, or the extraction liquid 6 by removing the magnetic rod 2 and the cover 3 from a container 7 containing the binding liquid 4, the probe cleaner 5, or the extraction liquid 6 after magnetism collection
"Diffusion" of the magnetic beads 1 into the binding liquid 4, the probe cleaner 5, or the extraction liquid 6 to be used next by moving the separated magnetic beads 1 into the binding liquid 4, the probe cleaner 5, or the extraction liquid 6 and removing the magnetic rod 2 while leaving the cover 3
Nucleic acid extraction is performed by combining and repeating these functions.

As illustrated in FIG. 1, a nucleic acid extraction step is roughly divided into the following three steps.

Step 11) Binding: For a biological sample containing a nucleic acid, the nucleic acid in the specimen and the magnetic beads 1 are bound by mixing the binding liquid 4 (configured with components such as a buffer for solubilizing a protein in the sample, a binding buffer containing a chaotropic salt, and the magnetic beads 1 for adsorbing nucleic acids under the presence of the chaotropic salt).

Step 11) A more detailed specific example of "binding" is as follows.

Step 11a) The liquid is "stirred" by vertical movement of the cover 3 to bind the nucleic acids to the magnetic beads 1.

Step 11b) The magnetic rod 2 is inserted into the cover 3, and the magnetic beads are collected on the magnetic rod 2 (cover 3). ("Magnetism Collection")

Step 11c) The magnetic rod 2 (cover 3) is removed from the container 7 to "separate" the magnetic beads 1 and the liquid.

Step 11d) The magnetic beads 1 are moved to the probe cleaner 5, the magnetic rod 2 is removed, and the magnetic beads 1 are "diffused" in the liquid.

Step 12) Washing: Mixing, magnetism collection, and separation of the magnetic beads 1 are performed as in step 11). For example, mixing and stirring of the magnetic bead 1 to which the nucleic acids are bound and the probe cleaner 5 and separation of the magnetic beads 1 and the probe cleaner 5 are performed to remove impurities such as proteins in the biological sample. In some cases, the cleaning is repeated a plurality of times.

Step 13) Extraction: The nucleic acids bound to the magnetic beads 1 are eluted into the extraction liquid 6 by mixing and stirring the magnetic beads 1 and the extraction liquid 6. The magnetic beads 1 are collected on the magnetic rod 2 and separated from the extraction liquid 6.

Step 13) A more detailed specific example of "extraction" is as follows.

Step 13a) The magnetic beads 1 are moved to the extraction liquid.

Step 13b) Stirring and magnetism collection of the magnetic beads 1 are performed as in step 11).

Step 13c) The magnetic beads and the extraction liquid are separated.

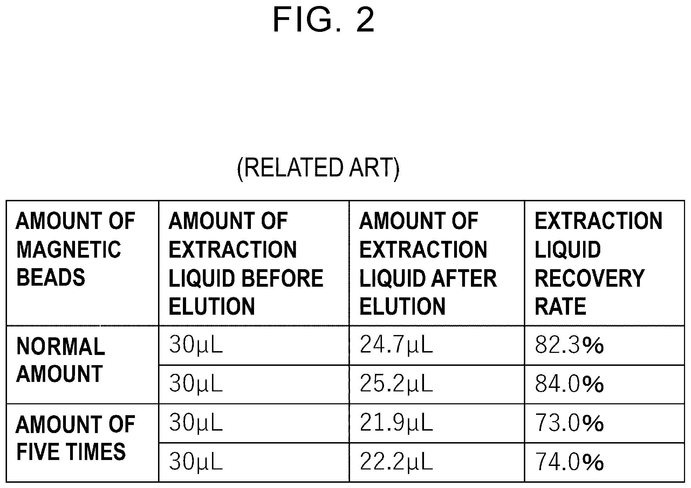

FIG. 2 illustrates a result obtained by experimentally examining an influence of the amount of the magnetic beads in the extraction step on a recovery amount of the extraction liquid in the related art. The amount of the magnetic beads is set to a amount of 5 times the normal amount as a second condition, extraction is performed with an extraction liquid of 30 µL, and the amount of the extraction liquid recovered in the container after being separated from the magnetic beads was measured. The experiment was performed under each condition of n = 2.

As a result, it was found that the liquid recovery amount was smaller than the normal amount under the condition that the amount of the magnetic beads was 5 times. This is because, when the magnetic beads and the extraction liquid are separated from each other, the extraction liquid adheres to the magnetic rod together with the magnetic beads and is lost.

The extraction liquid adhered to the magnetic rod together with the magnetic beads is referred to herein as a residual liquid.

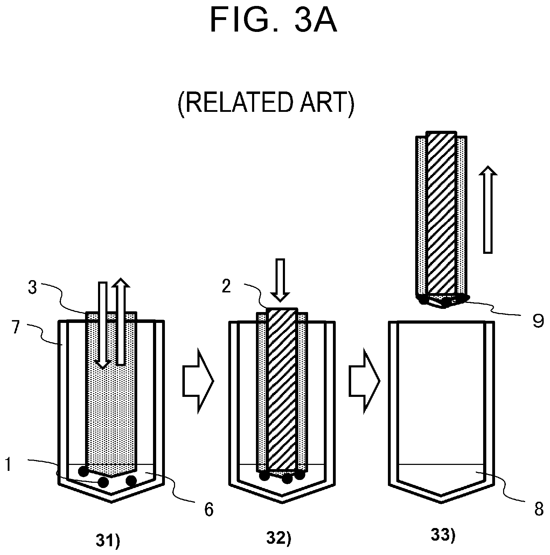

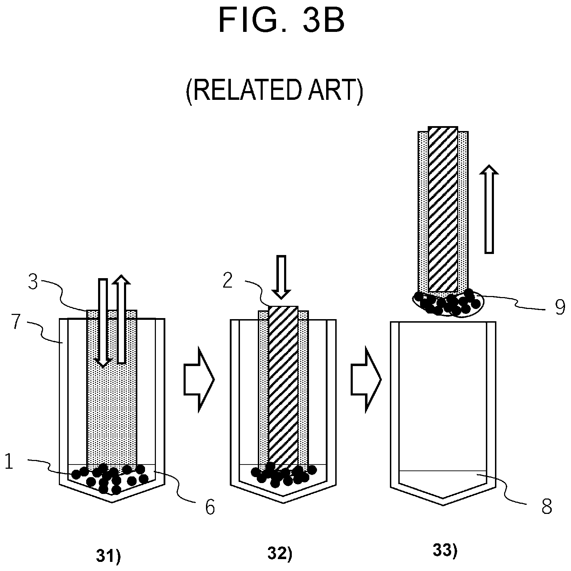

FIGS. 3A and 3B schematically illustrate a difference in the recovery amount of the extraction liquid depending on a difference in the amount of the magnetic beads 1 in this experiment.

FIG. 3A illustrates an extraction step in the case of using a normal amount of magnetic beads 1.

Step 31) The extraction liquid 6 and the magnetic beads 1 are mixed. When a mixed solution of the extraction liquid 6 and the magnetic beads 1 is mixed with the cover 3, the nucleic acids bound to the magnetic beads 1 are eluted into the extraction liquid 6.

Step 32) Next, when the magnetic rod 2 is inserted into the cover 3, the magnetic beads 1 are magnetically collected by the magnetic rod 2 through the cover 3.

Step 33) Finally, the magnetic beads 1 and the extraction liquid 6 are separated. When the magnetic rod 2 and the cover 3 are removed from the container 7, an extraction liquid 8 remains in the container 7.

The cover 3 may be omitted in the magnetism collection and separation steps. An amount of the extraction liquid 8 remaining in the container 7 is the recovery amount of the extraction liquid. A part of the extraction liquid adheres to the magnetic rod 2 or the cover 3 together with the magnetic beads 1 during the separation, and is lost as the residual liquid 9 of the extraction liquid.

FIG. 3B illustrates the case of using magnetic beads 1 in a amount of 5 times the normal amount. Operations in steps 31) to 33) are the same as those in FIG. 3A.

In step 33), the magnetic beads 1 are stacked on a surface of the magnetic rod 2 or the cover 3, and the extraction liquid enters the gap between the magnetic beads 1 to form the residual liquid 9 of the extraction liquid. As described above, the amount of the residual liquid 9 of the extraction liquid is larger than the condition of the normal amount of the magnetic beads 1, and as a result, the amount of the recovered extraction liquid 8 is smaller than the condition of the normal amount of the magnetic beads 1.

Hereinafter, in the embodiment of the present invention, an example of a liquid recovery device for additionally recovering the residual liquid 9 will be described. Descriptions of parts common to those of the related art described with reference to FIGS. 1, 2, 3A, and 3B may be omitted.

### <Embodiment 1>

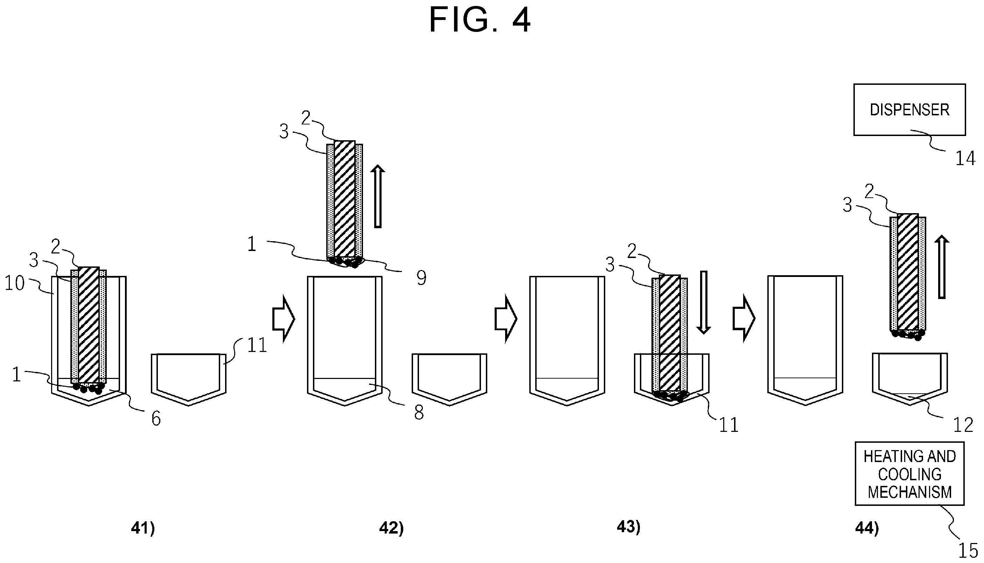

FIG. 4 illustrates a liquid recovery device according to Embodiment 1 of the present invention. FIG. 4 is an example of a device including a first container 10 for accommodating the magnetic beads 1 and the extraction liquid 6, and an empty second container 11.

The liquid recovery device includes a magnetic rod 2 that adsorbs the magnetic beads 1. Further, the cover 3 that covers the magnetic rod 2 may be provided. The cover 3 is detachable from the magnetic rod 2. The extraction liquid 6 is an extraction liquid for extracting nucleic acids from a biological sample. The magnetic beads 1 can adsorb and release nucleic acids.

The second container 11 is for recovering the residual liquid 9, and functions as a recovery region for recovering the extraction liquid as the residual liquid 9 from the magnetic rod 2 while the magnetic beads 1 are adhered thereto. That is, in the present embodiment, the recovery region is the second container 11 different from the first container 10.

The second container 11 has, for example, a container shape, but may be a flat surface or the like, and may have any shape as long as the second container 11 is a region for recovering the residual liquid 9.

Step 41) The liquid recovery device inserts, into the extraction liquid 6 in the first container 10, the cover 3 into which the magnetic rod 2 is inserted, and magnetically collects the magnetic beads 1. In this way, in a state where the cover 3 is attached to the magnetic rod 2, the magnetic rod 2 adsorbs the magnetic beads 1 through the cover 3. In the first container 10, after the nucleic acids are eluted from the magnetic bead 1 into the extraction liquid 6, the magnetic beads 1 in the extraction liquid 6 are magnetically collected by the magnetic rod 2. In this way, the liquid recovery device extracts nucleic acids in the first container 10.

Step 42) The magnetic beads 1 and the extraction liquid 6 are separated by removing, from the first container 10, the cover 3 into which the magnetic rod 2 is inserted. When the magnetic rod 2 is removed from the first container 10, the magnetic beads 1 and the extraction liquid 8 are separated, and the extraction liquid 8 that can be recovered in the first container 10 remains. In this way, the nucleic acids are released from the magnetic bead 1 by putting the magnetic rod 2 into the extraction liquid 6 and stirring the extraction liquid 6.

The extraction liquid 8 is also referred to as a "first recovery liquid". At this time, the extraction liquid adheres to the magnetic rod 2 as the residual liquid 9 together with the magnetic beads 1.

Step 43) Next, the magnetic rod 2 and the cover 3 are brought into contact with the second container 11. That is, the magnetic rod 2 to which the residual liquid 9 adheres is brought into contact with the second container 11.

Step 44) Finally, when the magnetic rod 2 is removed from the second container 11, the extraction liquid (residual liquid 9) adhering to the magnetic rod 2 or the cover 3 at that time moves to the second container 11 and can be recovered as an additional recovery liquid 12.

As described above, the liquid recovery device brings the magnetic rod 2 in a state of adsorbing the magnetic beads 1 into contact with the second container 11 (for example, the inside thereof, more specifically, an inner wall or a bottom surface), and recovers the residual liquid 9 as the additional recovery liquid 12.

Although not illustrated, a third container and the following containers may be further provided, and additional recovery may be similarly performed.

The additional recovery liquid 12 is equivalent to the extraction liquid 8 (first recovery liquid). The liquid recovery device may include a dispenser 14 as illustrated in a block diagram in step 44) of FIG. 4. The dispenser 14 recovers the residual liquid adhering to the second container 12, that is, the additional recovery liquid 12. It is also possible to integrate the extraction liquid 8 (first recovery liquid) and the additional recovery liquid 12 with the dispenser 14. By using the dispenser 14, the extraction liquid can be efficiently recovered.

In addition, after nucleic acid extraction, a QC step for measuring the concentration and size of the nucleic acid is generally performed. An amount of liquid used in the QC step is as small as about 1 µL to 20 µL in many cases. The additional recovery liquid 12 may be used in the QC step.

Although the cover 3 may be omitted, the use of the cover 3 that is detachable from the magnetic rod 2 makes it easier to remove the magnetic beads 1 from the magnetic rod 2.

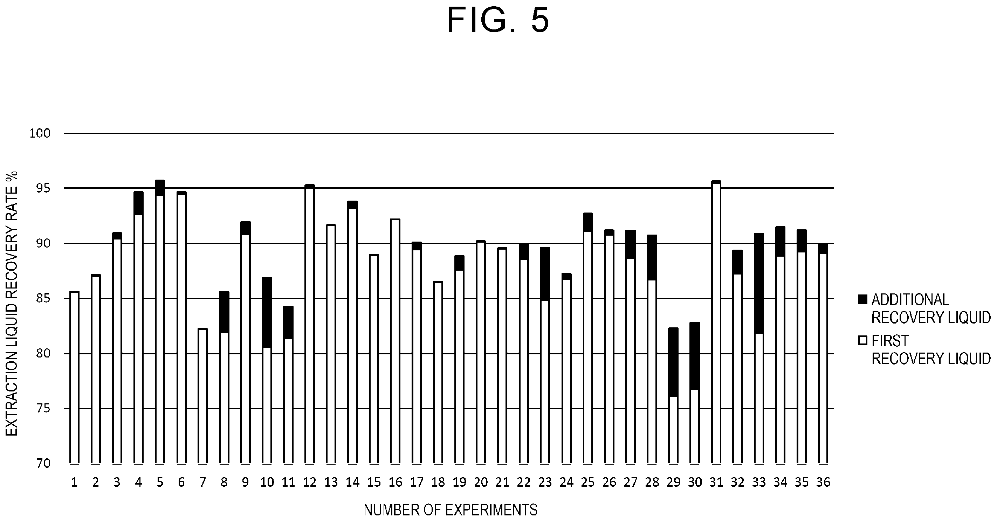

FIG. 5 illustrates a result of an experiment for verifying an effect of additional collection. When the number of experiments was n = 36, the elution was performed with an amount of the extraction liquid of 30 µL using magnetic beads in a amount of 5 times the normal amount. The amount of the extraction liquid 8 (first recovery liquid) and an amount of the additional recovery liquid 12 from the second container 11 were measured. Next, each amount of the recovery liquid was divided by 30 µL, multiplied by 100, and converted into a recovery rate of the extraction liquid, which was graphed.

As a result, it was confirmed that the extraction liquid could be additionally recovered in many experiments by the contact with the second container 11. In addition, it was found that a recovery rate of the additional recovery liquid 12 tended to increase when a recovery rate of the extraction liquid 8 (first recovery liquid) was low.

Further, the liquid recovery device may include a heating and cooling mechanism 15 as illustrated in a block diagram in step 44) of FIG. 4. The heating and cooling mechanism 15 is a mechanism for heating and/or cooling the region for recovering the residual liquid 9 (here, the second container 11) and the magnetic rod 2.

The heating and cooling mechanism 15 may heat the magnetic rod 2, may cool the second container 11, or may perform both of heating and cooling. Accordingly, the heating and cooling mechanism 15 makes a temperature of the magnetic rod 2 relatively higher than that of the second container 11. The heating and cooling method may be any method such as Peltier, air-cooling, or water-cooling.

In general, the viscosity of a liquid has temperature dependency, and the viscosity tends to decrease as the temperature increases. When the magnetic rod 2 or the cover 3 to which the residual liquid 9 adheres is brought into contact with the region for recovering the residual liquid 9, the temperature of the magnetic rod 2 or the cover 3 is set to be relatively higher than that of the region for recovering the residual liquid 9, so that the liquid can be easily moved from the higher temperature side to the lower temperature side. Therefore, the recovery of the additional recovery liquid 12 is promoted by heating the magnetic rod 2 or the cover 3 or cooling the region for recovering the residual liquid 9.

A material promotes the movement of the liquid form another viewpoint. Liquid movement is promoted at the time of contact by forming the magnetic rod 2 or the cover 3 with a hydrophobic material or coating the magnetic rod 2 or the cover 3 with a hydrophobic material, while forming the region for recovering the residual liquid 9 with a hydrophilic material or coating the magnetic rod 2 or the cover 3 with a hydrophilic material.

As described above, according to the liquid recovery device in Embodiment 1, the loss of separation between the magnetic beads 1 and the extraction liquid (residual liquid 9) is reduced.

Further, by using the second container 11 different from the first container 10, a shape of the first container 10 can be simplified.

### <Embodiment 2>

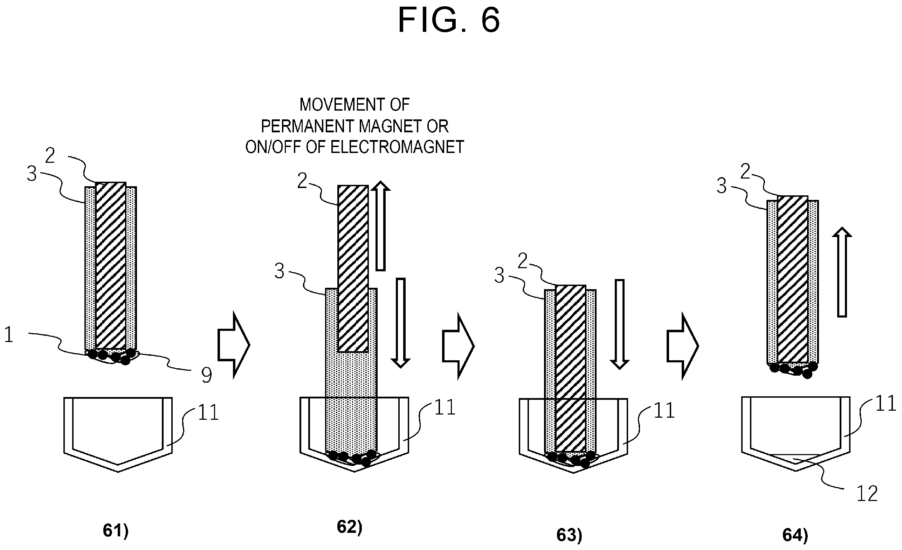

A method different from that of Embodiment 1 will be described with reference to FIG. 6 for an operation flow when the magnetic rod 2 to which the residual liquid 9 adheres is brought into contact with the region (here, the second container 11) for recovering the residual liquid 9. Hereinafter, description of parts common to Embodiment 1 may be omitted.

Step 61) At a point in time after the extraction liquid 8 (first recovery liquid) is recovered in the first container 10 (not illustrated in FIG. 6), the extraction liquid adheres to the magnetic rod 2 as the residual liquid 9 together with the magnetic beads 1 through the cover 3.

Step 62) The magnetic rod 2 is removed from the cover 3, and the cover 3 is brought into contact with the second container 11.

Step 63) Next, the magnetic rod 2 is inserted into the cover 3.

Step 64) Finally, the magnetic rod 2 and the cover 3 are removed from the second container 11. In this way, a part of the extraction liquid (residual liquid 9) adhering to the magnetic rod or the cover moves to the second container 11 and can be recovered as the additional recovery liquid 12.

For example, a permanent magnet can be used for the magnetic rod 2, but the same function can also be realized by using an electromagnet for the magnetic rod 2.

The electromagnet can switch on/off the magnetic force and change the strength of the magnetic force. An operation flow in the case of the electromagnet is a flow in which the electromagnet is turned on when the magnetic beads 1 are collected from the first container 10, the electromagnet is once turned off when the magnetic rod 2 comes into contact with the region (for example, the second container 11) for recovering the residual liquid 9, the electromagnet is switched on again, and the additional recovery liquid 12 adheres to the region for recovering the residual liquid 9.

### <Embodiment 3>

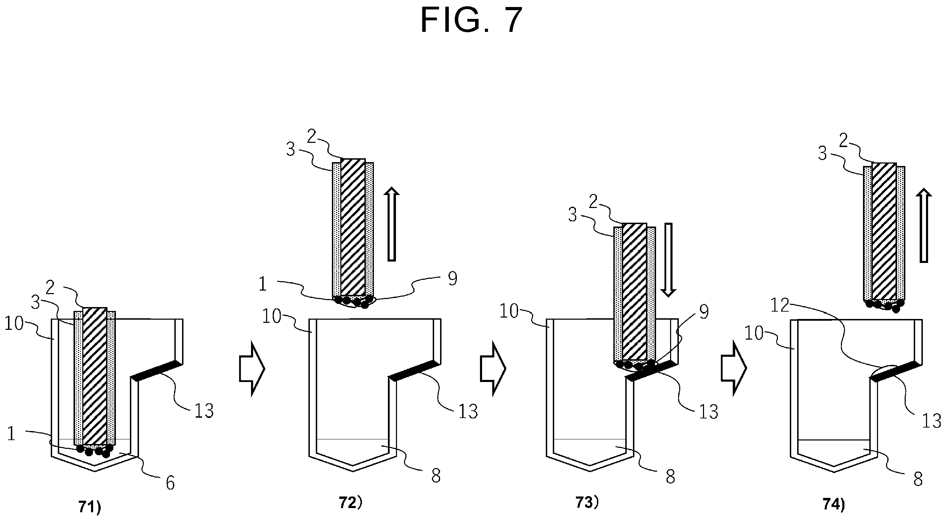

FIG. 7 illustrates a liquid recovery device according to Embodiment 3 of the present invention. Hereinafter, description of parts common to Embodiment 1 or 2 may be omitted.

FIG. 7 is an example of a device including a contact portion 13 which is a region for recovering the residual liquid 9 in a part of the first container 10 accommodating the magnetic beads 1 and the extraction liquid 6. The contact portion 13 is an example of a recovery region for recovering the residual liquid 9 from the magnetic rod 2 in a state of adsorbing the magnetic beads 1. The contact portion 13 may be a flat surface or may not be a flat surface. The contact portion 13 may have any shape as long as the contact portion 13 is a region for recovering the residual liquid.

Step 71) After the nucleic acids are eluted from the magnetic beads 1 into the extraction liquid 6 in the first container 10, the magnetic beads 1 in the extraction liquid 6 are magnetically collected by the magnetic rod 2. That is, the cover 3 into which the magnetic rod 2 is inserted is inserted into the extraction liquid 6 in the first container 10, and the magnetic beads 1 are magnetically collected.

Step 72) When the magnetic rod 2 is removed from the first container 10, the magnetic bead 1 and the extraction liquid 8 are separated from each other, and the extraction liquid 8 (first recovery liquid) that can be recovered remains in the first container 10. At this time, the extraction liquid adheres to the magnetic rod 2 as the residual liquid 9 together with the magnetic beads 1.

Step 73) Next, the magnetic rod 2 to which the residual liquid 9 adheres is brought into contact with the contact portion 13 in the first container 10.

Step 74) Finally, when the magnetic rod 2 is separated from the contact portion 13, the extraction liquid (residual liquid 9) adhering to the magnetic rod 2 or the cover 3 moves to the contact portion 13 and can be recovered as the additional recovery liquid 12.

Although not illustrated, a plurality of such contact portions 13 may be provided in the container, and the operations in step 73) and step 74) may be repeated. After the extraction liquid 8 (first recovery liquid) is separated, the magnetic rod 2 and the cover 3 may be moved along an inner wall of the first container 10 while being in contact with the inner wall to reach the contact portion 13.

In this way, by providing the contact portion 13 in the first container 10, the second container becomes unnecessary, and the overall configuration becomes simpler.

In the example of FIG. 7, the contact portion 13 includes a surface that is not parallel to a vertical direction, and in particular, the entire contact portion 13 is configured with the surface that is not parallel to the vertical direction. With such a configuration, a larger area of the contact portion 13 can be brought into contact with the extraction liquid collected in a bottom portion of the magnetic rod 2 by gravity, and the amount of the additional recovery liquid 12 increases.

### <Embodiment 4>

Further, the recovery efficiency of the extraction liquid can be increased by strengthening the magnetic force of the magnetic rod 2. The strength of the magnetic force can be changed according to a type, size, shape of the magnet, distance from an object, and the like.

When the magnetic rod 2 is brought into contact with a region (for example, the second container 11) in which the residual liquid 9 is recovered, by using the magnetic rod 2 having a stronger magnetic force, there is an effect of magnetically collecting the magnetic beads 1 firmly in the magnetic rod 2 and moving a larger amount of liquid with respect to the region in which the residual liquid 9 is recovered, and the additional recovery liquid 12 increases.

When the magnetic beads 1 are magnetically collected from the first container 10 after the nucleic acids are eluted from the magnetic beads 1 into the extraction liquid 6, the magnetic rod 2 having a stronger magnetic force may be used to reduce the amount of the residual liquid 9 adhering to the magnetic rod 2.

However, the magnetic force is not simply increased in all the steps, and it is preferable to use the magnetic rod 2 having a magnetic force suitable for each step of nucleic acid extraction. For example, when the magnetic force of the magnetic rod 2 is too strong, the magnetic beads 1 magnetically collected by the magnetic rod 2 are firmly adhered to each other, and thus even when the magnetic rod 2 is removed in a cleaning or elution process in the nucleic acid extraction step, the magnetic beads 1 are less likely to diffuse into the binding liquid 4, the probe cleaner 5, or the extraction liquid 6. On the other hand, when the magnetic beads 1 are magnetically collected from the binding liquid 4, the probe cleaner 5, or the extraction liquid 6, if the magnetic force is too weak, it is difficult to collect a sufficient amount of the magnetic beads 1 from the binding liquid 4, the probe cleaner 5, or the extraction liquid 6. Depending on the step, it is preferable to adjust the acting magnetic force by replacing the magnetic rod with the magnetic rod 2 having an appropriate magnetic force or adjusting a distance between the magnetic rod 2 and the magnetic bead 1 on which the magnetic force acts.

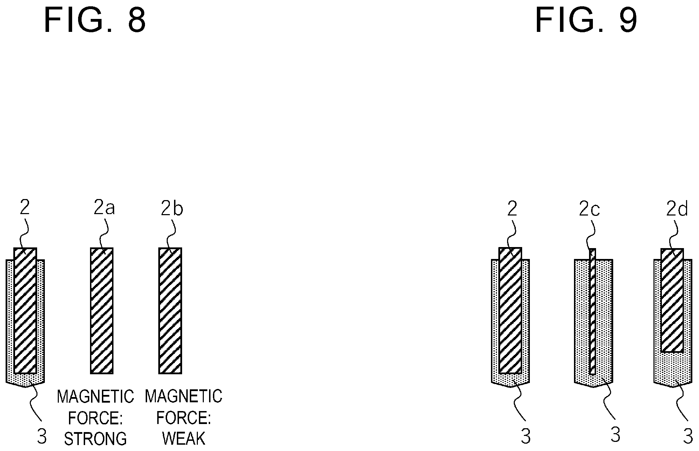

FIG. 8 illustrates a specific example of replacement of a magnetic rod. The liquid recovery device includes other magnetic rods 2a and 2b in addition to the magnetic rod 2. The magnetic rods 2a and 2b are different in magnetic force from the magnetic rod 2, and for example, the magnetic rod 2a is stronger in magnetic force than the magnetic rod 2 and the magnetic rod 2b is weaker in magnetic force than the magnetic rod 2. The liquid recovery device can change the magnetic force that acts on the magnetic beads 1 by replacing these magnetic rods, that is, by selectively using any of these magnetic rods according to the step.

FIG. 9 illustrates a specific example of adjustment of the distance between the magnetic rod 2 and the magnetic beads 1. The liquid recovery device includes other magnetic rods 2c and 2d in addition to the magnetic rod 2. The magnetic rod 2c is thinner than the magnetic rod 2 (that is, a radial dimension is small, and a diameter is small when the magnetic rod has a cylindrical shape). Therefore, when the same cover 3 is used, a distance between the magnetic rod 2c and the magnetic beads 1 is larger than the distance between the magnetic rod 2 and the magnetic beads 1, and the magnetic force that acts on the magnetic beads 1 can be changed according to the distance. Similarly, a magnetic rod having a larger radial dimension than the magnetic rod 2 may be used as long as the magnetic rod can be accommodated in the cover 3.

The magnetic rod 2d is shorter than the magnetic rod 2. Therefore, when the magnetic rod is inserted into the cover 3, a distance between a tip of the magnetic rod 2d and the magnetic bead 1 is larger than a distance between a tip of the magnetic rod 2 and the magnetic bead 1. Similarly, a magnetic rod longer than the magnetic rod 2 may be used as long as the magnetic rod can be accommodated in the cover 3.

In the example of FIG. 9, a distance between a tip of the magnetic rod and the magnetic beads 1 is changed by changing a length of the magnetic rod, but even when the length of the magnetic rod does not change (for example, when the same magnetic rod 2 is used), the distance between the tip of the magnetic rod 2 and the magnetic beads 1 can be changed by changing an insertion length of the magnetic rod 2 into the cover 3.

As described above, the liquid recovery device can change the magnetic force that acts on the magnetic beads 1 by adjusting the distance between the magnetic rod and the magnetic beads 1.

The same effect can also be obtained by the electromagnet. The electromagnet can switch on/off the magnetic force and change the strength of the magnetic force. In particular, the magnetic force can be strengthened by adjusting the magnetic force of the electromagnet when the magnetic beads 1 are magnetically collected from the first container 10 or when the magnetic rod 2 is brought into contact with a region (for example, the second container 11) in which the residual liquid 9 is recovered.

As described above, when the magnetic rod 2 includes the electromagnet, the liquid recovery device can change the magnetic force that acts on the magnetic beads 1 by changing a voltage applied to the magnetic rod 2.

In the above embodiments, an example has been described in which the residual liquid 9 is additionally recovered when the residual liquid 9 which is an extraction liquid adhering to the magnetic rod 2 or the cover 3 together with the magnetic beads 1 is present. The embodiments may be used in combination.

### Reference Signs List

1: magnetic bead
2, 2a to 2d: magnetic rod
3: cover
4: binding liquid
5: probe cleaner
6: extraction liquid
7: container
8: extraction liquid
9: residual liquid
10: first container
11: second container (recovery region)
12: additional recovery liquid
13: contact portion (recovery region)
14: dispenser
15: heating and cooling mechanism

## Claims

1. A liquid recovery device comprising:
a magnetic rod that adsorbs magnetic beads;
a first container that accommodates an extraction liquid for extracting nucleic acids, and the magnetic beads configured to adsorb and release the nucleic acids; and
a recovery region for recovering the extraction liquid as a residual liquid from the magnetic rod in a state of adsorbing the magnetic beads, wherein
the liquid recovery device extracts the nucleic acids in the first container, then brings the magnetic rod in the state of adsorbing the magnetic beads into contact with the recovery region, and recovers the residual liquid.

2. The liquid recovery device according to claim 1, further comprising:
a cover that covers the magnetic rod, wherein
the cover is detachable from the magnetic rod, and
the magnetic rod adsorbs the magnetic beads through the cover in a state where the cover is attached to the magnetic rod.

3. The liquid recovery device according to claim 1, wherein
the recovery region is a second container different from the first container.

4. The liquid recovery device according to claim 1, further comprising
a dispenser, wherein
the dispenser recovers the residual liquid adhering to the recovery region.

5. The liquid recovery device according to claim 1, further comprising
a heating and cooling mechanism, wherein
the heating and cooling mechanism heats the magnetic rod and/or cools the recovery region, thereby making a temperature of the magnetic rod relatively higher than that of the recovery region.

6. The liquid recovery device according to claim 1, wherein
the recovery region is provided as a contact portion in a part of the first container.

7. The liquid recovery device according to claim 1, further comprising
the plurality of magnetic rods, wherein
the liquid recovery device changes a magnetic force that acts on the magnetic beads by replacing the magnetic rod.

8. The liquid recovery device according to claim 1, wherein
the liquid recovery device changes a magnetic force that acts on the magnetic beads by adjusting a distance between the magnetic rod and the magnetic beads.

9. The liquid recovery device according to claim 1, wherein
the magnetic rod includes an electromagnet, and the liquid recovery device changes a magnetic force that acts on the magnetic beads by changing a voltage applied to the magnetic rod.

10. The liquid recovery device according to claim 6, wherein
the contact portion includes a surface that is not parallel to a vertical direction.
